# EUROPEAN PATENT APPLICATION

(11) **EP 4 603 576 A1**
(43) Date of publication of application: **20.08.2025**
(21) Application number: 23877634.8
(22) Date of filing: 10.10.2023
(51) Int. Cl.: C12N 5/00, C12N 5/0775, C12N 5/071, A61K 35/28, A61P 19/00

(54) **METHOD FOR PREPARING ORGANOID USING STEM CELL SELF-ORGANIZING ABILITY AND ORGANOID PREPARED BY SAME METHOD**

(30) Priority: 12.10.2022 KR 20220130869
(71) Applicant: Cell in Cells Co., Ltd, Jongno-gu, Seoul 03080 (KR)
(72) Inventor: CHO, Jae Jin, Seoul 06288 (KR)
(74) Representative: Mullholland, Lewis Paul
(86) International application number: PCT/KR2023/015546
(87) International publication number: WO 2024/080707

(57) **Abstract**

The present invention relates to a novel method for preparing an organoid using the self-assembly of spheroid. Particularly, the organoid prepared by the preparation method of the present invention has a cup-shaped morphology with a concave center, and thus, the following advantages may be obtained: nutrients and oxygen can be smoothly supplied to the center; and limitations in the size of organoid can be overcome.

## Description

### Technical Field

The present invention relates to a novel method for preparing an organoid-tissue module using the self-assembly of spheroid and an organoid-tissue module prepared by the above method. Particularly, the organoid-tissue module prepared by the preparation method of the present invention has a cup-shaped morphology with a concave center, and thus, the following advantages may be obtained: nutrients and oxygen can be smoothly supplied to the center; and limitations in the size of organoid can be overcome.

### Background Art

Tissue engineering aims to develop functional alternative materials for damaged tissues to restore or improve the functions of organs or tissues (Griffith & Naughton, 2002; Langer & Vacanti, 1993). In order to develop such functional alternative materials, research on organoids (organ analogs) that can mimic human organ tissues is very important. When stem cells are cultured using a three-dimensional culture method, organoids, which are 3D cell aggregates composed of multiple cells, are formed through self-renewal and self-organization (Kaushik *et al,* 2018). Since the formed organoids can mimic the structural and functional characteristics of living tissues, they can be utilized not only for tissue regeneration therapy, but also as miniaturized organ models for patient-tailored disease modeling or drug screening systems (Kaushik *et al.,* 2018).

Embryonic stem cells (ESC), induced pluripotent stem cells (iPSC), and adult stem cells (ASC) are used as cell sources for organoids. In particular, mesenchymal stem cells (MSCs), one type of adult stem cell, are attracting attention as a cell source for organoids because they can be easily isolated and cultured from various adult tissues such as bone marrow and adipose tissue (Jiang *et al,* 2002; Pittenger *et al,* 1999; Wang *et al,* 2014). In order to culture these stem cells in three dimensions, a structural support similar to the extracellular matrix is required. Matrigel is the most commonly used support, but its components have not been fully characterized and it contains several growth factors that are difficult to quantify, making it difficult to consistently control the environment surrounding the cells each time the organoids are cultured. Alternatively, 3D synthetic supports can be also used as biomaterials such as hydrogel and polyethylene glycol. However, artificial supports have the disadvantage that they cannot perfectly reproduce the extracellular matrix of living tissue (Hofer & Lutolf, 2021; Khademhosseini & Langer, 2016). Therefore, the demand for producing organoids without the help of artificial supports is increasing.

Embryology-based tissue engineering attempts to overcome the dependence on these artificial supports by utilizing the ability of cells to synthesize their own extracellular matrix under specific conditions (Burdis & Kelly, 2021). When cells attach to the extracellular matrix they synthesize themselves, they enable the interaction between cells and cells and between cells and extracellular matrix more similar to actual tissues, and through this interaction, biological processes such as self-assembly and self-organization occur. Self-assembly and self-organization processes can be induced through cell aggregation technology. Most cell aggregation technologies are based on physical external forces such as centrifugal force and electromagnetic fields, and most have a structure close to a sphere. Spherical organoids produced by these technologies are structurally limited in size because oxygen and nutrients are not sufficiently supplied to the center. Therefore, a long-term culture time is required to obtain large organoids, and an increase in culture time makes it difficult to control the cell composition of the organoids.

Accordingly, in order to solve the above problems, the present inventors developed a method for preparing organoids that are not limited in size through the self-assembly and self-organization ability of stem cells, and isolated human mesenchymal stem cells (hMSCs) from human adipose tissue, then cultured them in two dimensions, and produced millimeter-sized organoid-tissue modules composed of mesenchymal stem cells using a three-dimensional culture technology. Based on the above, the present inventors completed the present invention.

Through this, the present inventors confirmed that the cup-shaped cartilage-organoids of millimeter size were prepared in a chondrocyte differentiation environment, and that the organoids were transplanted to the cartilage of damaged joints to have the effect of cartilage regeneration.

### Detailed Description of Invention

### Technical Problem

The present invention provides a method for preparing organoids of several millimeters in size by mixing and culturing spheroids with different sizes and utilizing the self-assembly ability of spheroids. The method of the present invention simplifies the complex method for preparing organoids and provides an organoid having a cup-shaped morphology capable of supplying nutrients and oxygen to the central region.

### Solution to Problem

In order to achieve the object described above, the present invention provides a method for preparing an organoid-tissue module by mixing and culturing spheroids with different diameters.

In addition, the present invention provides an organoid-tissue module prepared by the above preparation method.

In addition, the present invention provides a pharmaceutical composition for preventing or treating cartilage disease, comprising an organoid-tissue module prepared by the above preparation method.

In the above preparation method, the spheroid may be characterized by having a diameter of 10 to 1,000 µm, and preferably may have a diameter of 50 to 500 µm.

In the above preparation method, spheroids having a diameter of 200 µm or less may account for 60% or more of the total spheroids, preferably 70% or more, and more preferably 80% or more.

In addition, in the above preparation method, spheroids having a diameter of more than 200 µm may account for 40% or less of the total spheroids, preferably 30% or less, and more preferably 20% or less.

In addition, in the above preparation method, the number of spheroids having a diameter of 200 µm or less may be greater than the number of spheroids having a diameter of more than 200 µm.

In addition, in the above preparation method, the occupied space of the spheroids having a diameter of 200 µm or less may be greater than the occupied space of the spheroids having a diameter of more than 200 µm.

In addition, in the above preparation method, the space not occupied by the spheroids may account for 5 to 40% of the total space for culturing the spheroids, preferably, the space not occupied by the spheroids may account for 10 to 35% of the total space for culturing the spheroids, and more preferably, the space may account for 15 to 30% of the total space.

In addition, in the above preparation method, the organoid-tissue module may occupy 10 to 35% of the culture area, and the organoid-tissue module may have an average diameter of 2 to 4 mm.

In addition, in the above preparation method, the spheroid may be obtained by culturing stem cells or undifferentiated cells, preferably, the stem cell and undifferentiated cell may be selected from the group consisting of an embryonic stem cell, a dedifferentiated stem cell, an adult stem cell, and a mesenchymal stem cell, and more preferably, the stem cell may be a human adipose-derived mesenchymal stem cell (hMSC).

In addition, in the above preparation method, the organoid-tissue module may have a cup-shaped morphology.

In addition, the above preparation method may not include a scaffold and an artificial material.

### Effects of Invention

The present invention provides a technology for preparing an organoid-tissue module and tissue complex free from a scaffold and a xenograft. The organoid-tissue module prepared by the preparation method of the present invention has a cup-shaped morphology, and thus, it does not have problems with oxygen and nutrient supply, and an organoid-tissue module can be easily prepared in a short period of time by utilizing the self-assembly ability of spheroids.

In addition, the organoid prepared by the preparation method of the present invention retains the characteristics of stem cells and can be used for the treatment of various diseases including cartilage disease.

### Brief Description of Drawings

Figure 1 is a schematic diagram showing the principle of the technology for producing an organoid-tissue module (TM).
Figure 2 is a photograph and graph showing the morphology of human mesenchymal stem cells (hMSCs) and cell division ability according to passage culture.
Figure 3 is a graph showing the stem cell surface positive marker of hMSCs using FACS.
Figure 4 is a graph showing the stem cell surface negative marker of hMSCs using FACS.
Figure 5 is a photograph obtained by a microscope confirming that hMSCs have differentiation ability characteristics.
Figure 6 is a photograph showing a spheroid mixture that failed to generate an organoid-tissue module.
Figure 7 is a photograph showing a spheroid mixture that successfully produced an organoid-tissue module.
Figure 8 is a schematic diagram and hourly photographs of a time-lapse image recording the process of forming organoid-tissue modules for 72 hours.
Figure 9 is a photograph showing the process of small-sized spheroids being attracted and fused around a large-sized spheroid during the process of forming organoid-tissue modules.
Figure 10 is a graph showing the size distribution of spheroids produced for the production of organoid-tissue modules.
Figure 11 is a graph showing the average diameter of spheroids belonging to each range, classified by diameter size of 200 µm or less and diameter size of more than 200 µm among the spheroid mixtures that successfully produced organoid-tissue modules for successful production of organoid-tissue modules.
Figure 12 is a graph showing the distribution of the number of spheroids according to the size of mixed spheroids from which the organoid-tissue module was produced and mixed spheroids from which the organoid-tissue module was not produced.
Figure 13 is a graph showing the distribution of the occupied space of the spheroids according to the size of mixed spheroids from which the organoid-tissue module was produced and mixed spheroids from which the organoid-tissue module was not produced.
Figure 14 is a graph showing the distribution of empty space not occupied by mixed spheroids from which the organoid-tissue module was produced and mixed spheroids from which the organoid-tissue module was not produced.
Figure 15 is a graph showing the average movement speed of spheroids over time during the process of forming organoid-tissue modules according to self-organization ability.
Figure 16 is a graph showing the deviation of the migration distance and average speed of spheroids over time during the process of forming organoid-tissue modules according to self-organization ability.
Figure 17 is a graph showing the trajectory of spheroids during the process of forming organoid-tissue modules according to self-organization ability.
Figure 18 is a graph showing the circularity, speed over time, and time to reach the initiation stage, condensation stage, and lifting and folding stage according to the organoid-tissue module formation stage.
Figure 19 is a photograph and graph showing that the size of spheroids changes when the number of cells is adjusted.
Figure 20 is a photograph showing the effect of the mixing ratio of two types of spheroids with different sizes on the formation of organoid-tissue modules.
Figure 21 is a photograph showing whether organoid-tissue modules are generated according to the ratio of the area not occupied by mixed spheroids relative to the total culture area.
Figure 22 is a photograph showing that the self-organization and cup-shaped morphology of organoid-tissue modules produced with two types of spheroids with different sizes are maintained.
Figure 23 is a photograph showing the process of fusion of two types of spheroids with different sizes.
Figure 24 is a photograph of a fluorescently stained organoid-tissue module taken with a confocal microscope.
Figure 25 is a photograph and graph showing the results of confirming the stem cell marker and differentiation ability of cells isolated from organoid-tissue modules.
Figure 26 is a photograph and graph showing the results of comparing organoid-tissue modules with spherical cell aggregates to show that they are resistant to apoptosis.
Figure 27 is a photograph showing the expression and location of type 2 collagen (type II collagen) in organoid-tissue modules in which cartilage differentiation was induced as confirmed by immunohistological staining.
Figure 28 is a photograph and graph showing the expression of aggrecan and the distribution of nuclei stained with SYTO16 by 3D clearing of organoid-tissue modules in which cartilage differentiation was induced.
Figure 29 is a graph showing the content of glycosaminoglycan (GAG), a cartilage extracellular matrix, in organoid-tissue modules in which cartilage differentiation was induced.
Figure 30 is a photograph showing the results of histological analysis by applying various staining techniques to organoid-tissue modules in which cartilage differentiation was induced.
Figure 31 is a photograph showing the results of histological analysis and the efficacy of transplantation of organoid-tissue modules in which cartilage differentiation was induced into rabbit cartilage.

### Best Mode for Carrying out the Invention

Hereinafter, with reference to the accompanying drawings, embodiments and examples of the present invention will be described in detail so that those of ordinary skill in the art to which the present invention belongs can easily practice the present invention. However, the present invention may be implemented in various forms and is not limited to the embodiments and examples described herein.

Throughout the present specification, when a certain part "includes" a certain component, it means that other components may be further included, rather than excluding other components, unless otherwise stated.

The method for preparing an organoid-tissue module of the present invention comprises the first step of isolating human mesenchymal stem cells (hMSCs) from human fat, culturing them in two dimensions, and using a three-dimensional culture technology to produce spheroids, and the second step of mixing and culturing spheroids with various sizes to produce an organoid-tissue module, which is a cup-shaped organoid.

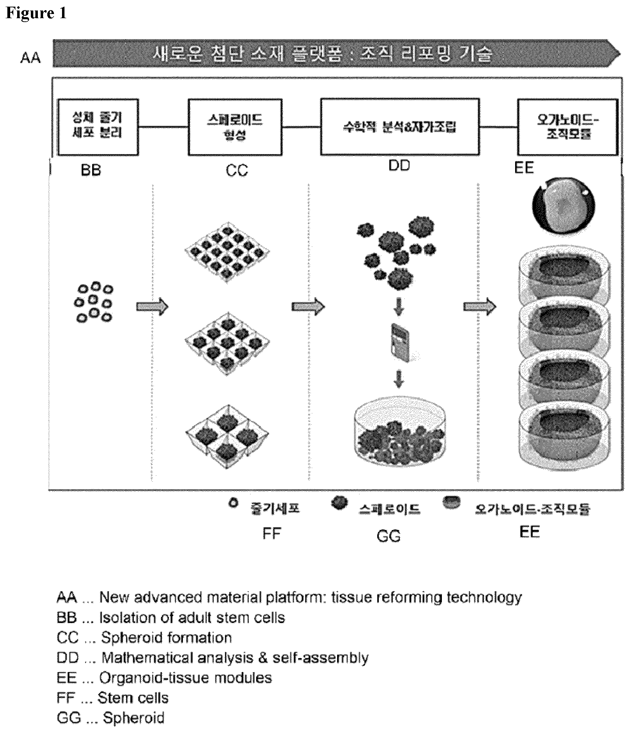

A schematic diagram of the present invention for producing an organoid of several millimeters in size from a single cell is shown in Figure 1.

The above "two-dimensional culture" refers to culturing cells in a monolayer in any type of culture vessel, such as a cell culture flask or a flat petri dish.

The above "three-dimensional culture" means that cells are cultured while interacting with all three dimensions surrounding them, and have the characteristic of being able to grow in all directions.

The above "organoid" refers to a tissue mimic that is a three-dimensionally cultured stem cell and has a form similar to actual human tissue and organs through self-organization.

The above "organoid-tissue module (TM)" refers to an organoid that has not differentiated into a specific organ tissue produced through self-organization of a spheroid.

The above "cup shape" refers to a form having a concave groove in the middle.

In the present invention, the "stem cell" includes an adult stem cell, preferably, it may be a "mesenchymal stem cell", and more preferably, it may be a "human adipose-derived mesenchymal stem cell."

Hereinafter, the present invention will be described in more detail through the examples, but the following examples are for illustrative purposes only and are not intended to limit the scope of the present invention.

### [Example 1]

### Isolation of mesenchymal stem cells (hMSCs) from adipose tissue

Human adipose-derived mesenchymal stem cells (human mesenchymal stem cells, hMSCs) with relatively easy isolation and high growth rate were used as the cell source for the production of the organoid-tissue module of the present invention (Meligy et al., 2012). The isolation of hMSCs was performed with the approval of the Institutional Review Board of Seoul National University Dental Hospital.

The prepared human derived adipose tissue was placed in Hanks^{®} Balanced Salt solution (HBSS) containing 0.1% collagenase I, and shaken at 37°C for 30 minutes to isolate the cells, and then, the cells were washed and filtered through a 100 µm nylon mesh. Thereafter, the cells were cultured in a 5% CO₂ incubator at 37 °C with nutrients supplied with Dulbecco's modified Eagles medium (DMEM) containing 10% fetal bovine serum (FBS) and 1X antibiotic-antimycotic solution (AA).

### [Example 2]

### Verification of isolated mesenchymal stem cells (hMSCs)

The hMSCs isolated as described above have characteristics such as adherent form, multipotency, and expression of surface-specific antigens (Dominici et al., 2006). By confirming these characteristics, it was verified that the cells isolated in Example 1 above were hMSCs.

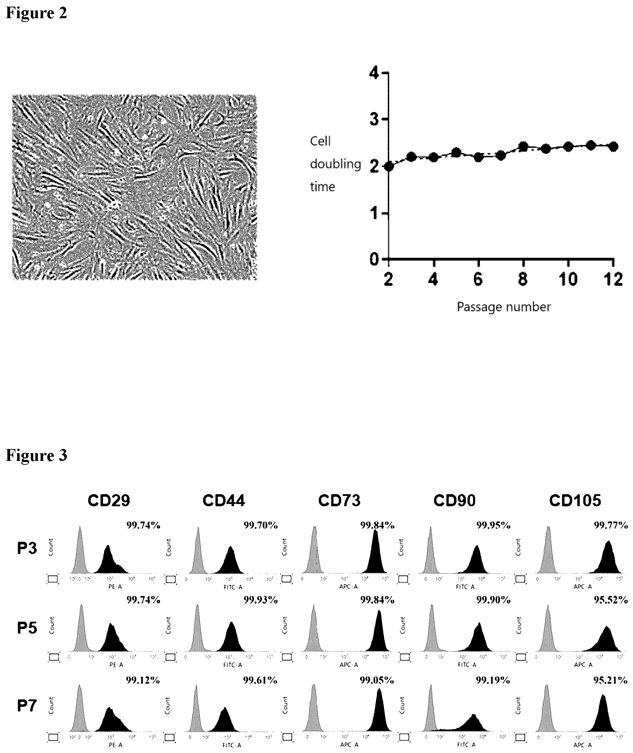

As a result of confirming the morphology and distribution characteristics of the above cells, as shown in Figure 2, the cells showed a uniform spindle-like shape attached and growing on a plastic culture dish, and the period for doubling the number of cells was maintained at a constant 2 days from the 2nd passage to the 12th passage.

In addition, as a means of confirming whether the characteristics of hMSCs were well maintained through passage, the expression of stem cell surface markers was confirmed through fluorescence. In the 3rd, 5th, and 7th passages, the cells were reacted with antibodies against each fluorescently labeled CD14, CD29, CD31, CD34, CD44, CD73, CD90, HLA-DR, CD45, CD79α, CD105, and CD117 at 4 °C for 1 hour. Thereafter, the cells were washed with Dulbecco's phosphate buffered saline (DPBS) containing 2% FBS, and the expression level compared to the isotype mouse IgG control group was analyzed by FACS.

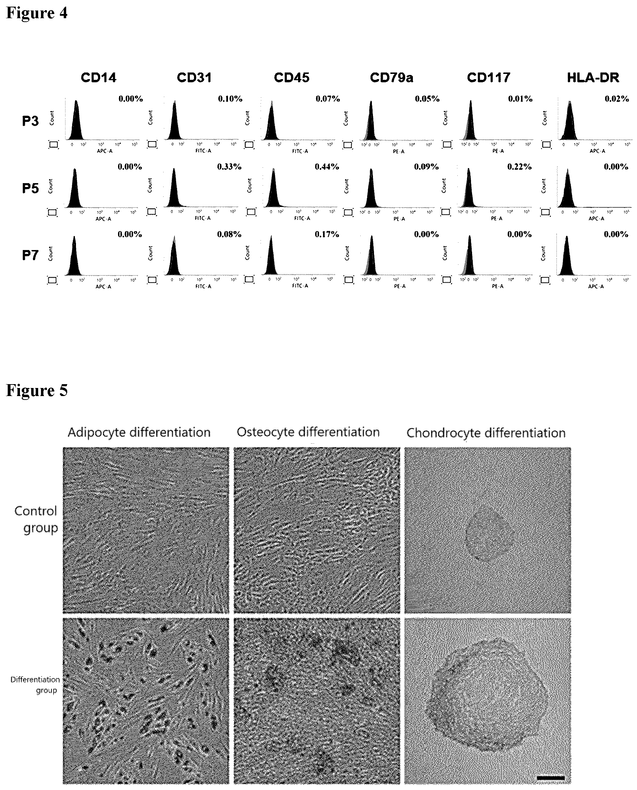

As a result of the test, 95% or more of the cells expressed positive markers of CD29, CD44, CD73, CD90, and CD105 as shown in Figure 3, and the expression of negative markers of CD14, CD31, CD45, CD79α, CD117, and HLA-DR was confirmed to be 2% or less as shown in Figure 4.

### [Example 3]

### Confirmation of osteocyte differentiation ability of isolated mesenchymal stem cells (hMSCs)

In order to confirm the osteocyte differentiation ability of hMSCs, 9.5 x 10⁴ cells were placed in a 6-well plate and cultured in a culture medium prepared by adding 10 nM dexamethasone, 50 µg/mL ascorbic acid, 10 mM β-glycerolphosphate, 1 mM dibutyryl-cAMP, 10% FBS, and 1X AA to an alpha minimum essential medium (α MEM) medium. After 4 days, all culture media were removed and cultured in a 5% CO₂ incubator at 37 °C for 14 days while replacing the culture media twice a week with a medium without dibutyryl-cAMP, to induce differentiation of hMSCs into osteocytes.

Thereafter, alizarin red S staining was performed to visually confirm osteocyte differentiation. The culture solution of the plate where differentiation was completed was removed and washed once with DPBS, and then 1 mL of 4% paraformaldehyde (PFA) was added, and the cells were fixed at room temperature. The fixative was removed, washed with DPBS, and 2 mL of alizarin red S solution diluted to 1% using distilled water (DW) was dispensed and reacted at room temperature for 15 minutes. Thereafter, it was washed 5 times with DW, and then 1 mL of DW was dispensed. Thereafter, as shown in Figure 5, differentiation into osteocytes (osteogenic differentiation) was confirmed using a microscope (Olympus).

### [Example 4]

### Confirmation of adipocyte differentiation ability of isolated mesenchymal stem cells (hMSCs)

In order to confirm the adipocyte differentiation ability, in the same manner as in Example 3 above, 9.5 x 10⁴ cells were placed in a 6-well plate and cultured in a 5% CO₂ incubator at 37 °C for 14 days while replacing the culture media twice a week with a culture medium prepared by adding 0.5 mM isobutylmethylxanthine (3-isobutyl-1-methylxanthine, IBMX), 100 nM dexamethasone, 100 µM indomethacin (INDO), 10 µg/mL insulin, 10% FBS, and 1X AA to high glucose DMEM. In order to confirm differentiation, the culture solution of the plate where differentiation was completed was removed, the cells were washed once with DPBS and then fixed with 4% PFA at room temperature.

Thereafter, in order to visually confirm differentiation, 1 mL of a solution prepared by dissolving 0.2 g of oil red O powder in 40 mL of isopropanol and filtering through a 0.22 µm filter was added, reacted for 15 minutes at room temperature, and washed 5 times with DW. Thereafter, as shown in Figure 5, differentiation into adipocytes (adipogenic differentiation) was confirmed using a microscope (Olympus).

### [Example 5]

### Confirmation of chondrocyte differentiation ability of isolated mesenchymal stem cells (hMSCs)

In order to confirm the chondrocyte differentiation ability, in the same manner as in Example 3 above, 9.5 x 10⁴ cells were placed in a 6-well plate and cultured in a 5% CO₂ incubator at 37 °C for 21 days by adding 10 ng/mL of transforming growth factor (TGF-β3) to a culture medium prepared by adding 1X insulin transferrin selenium premix (ITS), 50 ng/mL ascorbic acid, 40 µg/mL L-proline, 100 nM dexamethasone, etc., 10% FBS, and 1X AA to α MEM. Thereafter, the culture solution of the plate where differentiation was completed was removed, and the cells were fixed with 4% PFA at room temperature.

Once the cells were fixed, the cells were washed with DPBS, and the fixed cells were placed in OCT compound and frozen in an ultra-low temperature freezer (-80 °C) for one day. The frozen cells were sectioned to 8 µm thickness using a cryocut microtome and washed with DW. A 1% alcian blue solution prepared using alcian blue reagent was added to the sections and stained at room temperature for 30 minutes, then washed with a 0.1 N HCl solution, and washed with DW. Thereafter, as shown in Figure 5, differentiation into chondrocytes (chondrogenic differentiation) was confirmed using a microscope (Olympus).

As a result of confirming the differentiation ability through Examples 3 to 5 above, it was confirmed that the purity of the cells isolated from human fat by the technology of the present invention was very high, and it was confirmed that they had characteristics as stem cells even during long-term culture.

### [Example 6]

### Prior study for producing organoid-tissue modules

Generally, long-term culture is required to produce organoids, and when the size increases, the supply of oxygen or nutrients to the cells located in the center of the spherical organoids is limited, which increases the possibility of apoptosis. Therefore, we sought to find conditions for producing organoid-tissue modules having a cup-shaped morphology to ensure smooth supply of oxygen and nutrients to the center. After finding the conditions for producing organoid-tissue modules, we sought to provide a differentiation environment during the process of producing organoid-tissue modules to produce organoids that had increased sizes to several millimeters in a short period of time.

Using the hMSCs produced and confirmed in the above example, spheroids with various sizes were produced for producing organoid-tissue modules. 10,000 or 15,000 hMSCs were placed in each well of a 96-well flat or U-bottom plate and centrifuged at 500 x g for 5 minutes to induce forced aggregation. The cells were cultured, and randomly forced aggregated cell aggregates were cultured to produce spheroids with various sizes.

Large and small hMSC spheroids with a diameter of 50 to 500 µm were formed in each well, and all spheroids formed in the 96-well plate were collected. The spheroids were centrifuged to remove the supernatant, then resuspended in 200 µl of medium, and transferred to one well of an ultra-low attachment 96-well plate. They were placed in a 37°C incubator to induce self-assembly.

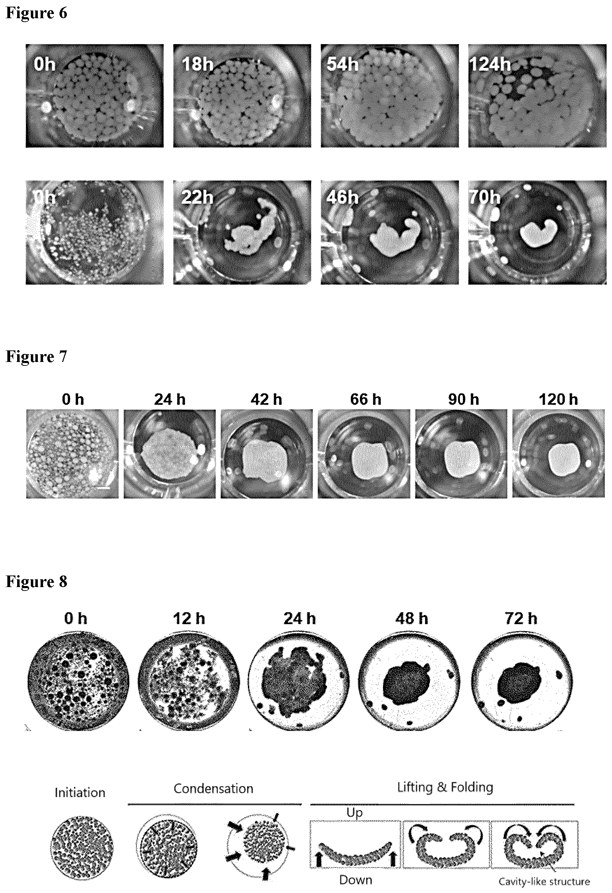

The generated spheroids ranged in size depending on the condition of the bottom of the 96-well plate used (flat bottom or round bottom). As shown in Figure 6, there were wells where self-assembly did not occur, and as shown in Figure 7, there were wells containing organoid-tissue modules with a unique structure that self-assembled and had a concave groove in the center. The unique self-assembly structure with a cup shape of 2 to 4 mm in diameter was referred to as an organoid-tissue module.

### [Example 7]

### Image analysis of organoid-tissue module generation of spheroid

The spheroids were mixed and transferred to a plate with a designated area (e.g., a 96-well plate), and time-lapse images were taken for 72 hours from the time point (0 hours) when the spheroids sank to the bottom of the well. Each time-lapse image was combined to create a video of the spheroid self-assembly process, and then the images were analyzed. As a result, as shown in Figure 8, it was confirmed that the spheroids moved dynamically and went through the initiation stage, condensation stage, and lifting and folding stage to generate organoid-tissue modules having a cup-shaped morphology.

The time point (0 hours) when the spheroids sank to the bottom of the well was referred to as the initiation stage, the stage in which each spheroid moved to the center and aggregated was referred to as the condensation stage, and the stage in which a cup shape was formed was referred to as the lifting and folding stage. In the initiation stage, each spheroid had a distinct boundary, and in the condensation stage, the spheroids moved to the center, fused with neighboring spheroids, and continuously contracted toward the core. In the lifting and folding stage, the edges of the organoid-tissue module were lifted and folded upward against gravity, to generate organoid-tissue modules having a cup-shaped morphology.

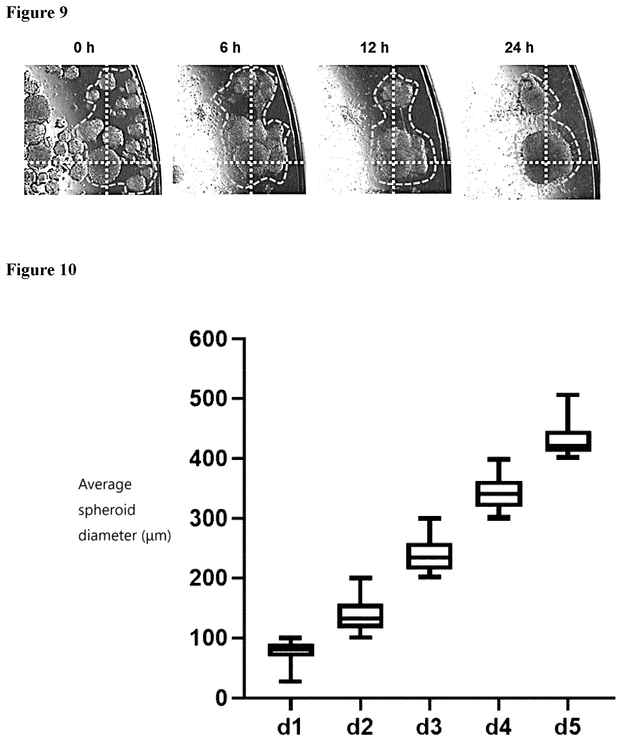

As shown in Figure 9, it was confirmed that when large and small spheroids aggregated, small spheroids were attracted to large spheroids and clumped together. These results indicate that spheroid size and the size-dependent ratio are important for inducing self-organization for organoid-tissue modules.

### [Example 8]

### Size analysis of spheroids for producing organoid-tissue modules

In order to confirm the spheroid mixing conditions for producing organoid-tissue modules, the initiation stage images from wells where organoid-tissue module production was successful and wells where it failed were analyzed.

Using ImageJ, the diameters of measurable spheroids in the initiation stage were obtained, and the spheroids belonging to each size range were classified as shown in Table 1 below and Figure 10.

**[Table 1]**

| | Spheroid diameter (µm) | Average spheroid diameter (µm) |
|---|---|---|
| d1 | 100 or less | 80 |
| d2 | 100 to 200 | 140 |
| d3 | 200 to 300 | 250 |
| d4 | 300 to 400 | 330 |
| d5 | 400 to 500 | 430 |

The average diameter of spheroids belonging to each size range was 80 µm for 100 µm or less, 140 µm for 100 to 200 µm, 250 µm for 200 to 300 µm, 330 µm for 300 to 400 µm, and 430 µm for 400 to 500 µm.

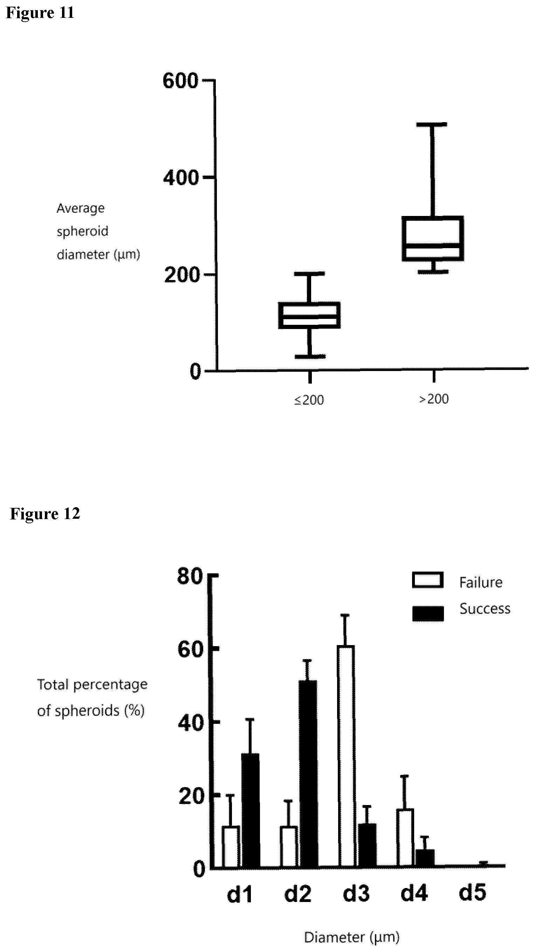

When analyzing the spheroid size of the well from which the organoid-tissue module was generated, as shown in Figure 11, the average diameter of the small spheroids with a diameter of 200 µm or less was 110 µm, and the average diameter of the large spheroids with a diameter of more than 200 µm was 270 µm.

### [Example 9]

### Analysis of number distribution of spheroids according to their sizes for production of organoid-tissue modules

The results of analyzing the number of spheroids according to their sizes that constituted the well (Success) from which the organoid-tissue module was successfully produced are shown in Table 2 below and Figure 12.

**[Table 2]**

| | Spheroid diameter (µm) | Number (%) |
|---|---|---|
| d1 | 100 or less | 31 |
| d2 | 100 to 200 | 51 |
| d3 | 200 to 300 | 12 |
| d4 | 300 to 400 | 5 |
| d5 | 400 to 500 | 1 |

The results of analyzing the number of spheroids of various sizes that constituted the well (Failure) from which the organoid-tissue module was not produced are shown in Table 3 below and Figure 12.

**[Table 3]**

| | Spheroid diameter (µm) | Number (%) |
|---|---|---|
| d1 | 100 or less | 12 |
| d2 | 100 to 200 | 11 |
| d3 | 200 to 300 | 61 |
| d4 | 300 to 400 | 16 |
| d5 | 400 to 500 | 0 |

As shown in Tables 2 and 3 above, when the spheroid diameters mixed in the organoid-tissue module initiation stage were of various sizes, from 50 to 500 µm, it was confirmed that organoid-tissue modules were not produced when 23% of spheroids had a diameter of 200 µm or less, and that organoid-tissue modules were produced when 82% of spheroids had a diameter of 200 µm or less.

In addition, it was confirmed that organoid-tissue modules were not produced when 77% of spheroids had a diameter of more than 200 µm, and that organoid-tissue modules were produced when 18% of spheroids had a diameter of more than 200 µm.

In addition, it was confirmed that organoid-tissue modules were produced when the number of spheroids having a diameter of 200 µm or less was greater than the number of spheroids having a diameter of more than 200 µm.

### [Example 10]

### Analysis of space occupancy ratio of spheroids for production of organoid-tissue modules

In order to confirm the conditions for the production of organoid-tissue modules, the occupied space and remaining space of spheroids according to their sizes were analyzed.

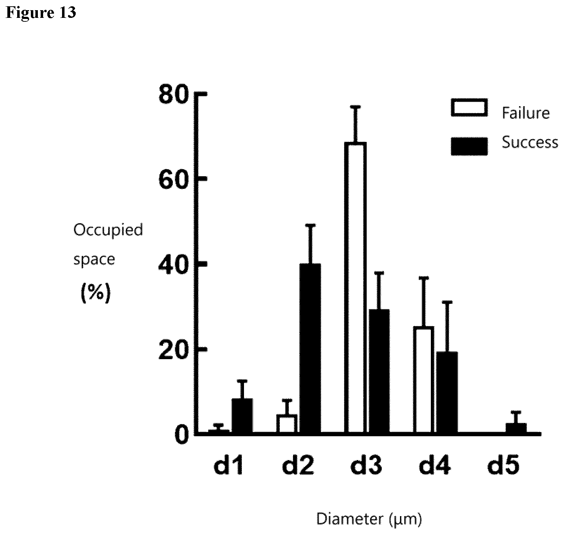

The results of analyzing the occupied space of spheroids according to their sizes that constituted the well (Success) from which the organoid-tissue module was successfully produced are shown in Table 4 below and Figure 13. The occupied space is expressed as a percentage of the sum of the spaces occupied by spheroids belonging to the following size ranges relative to the sum of the spaces occupied by all spheroids.

The space occupied by individual spheroids was calculated using the formula π x r² from the radius (r) derived from ImageJ analysis.

**[Table 4]**

| | Spheroid diameter (µm) | Average occupied space (%) |
|---|---|---|
| d1 | 100 or less | 8 |
| d2 | 100 to 200 | 40 |
| d3 | 200 to 300 | 29 |
| d4 | 300 to 400 | 20 |
| d5 | 400 to 500 | 3 |

The results of analyzing the occupied space of spheroids according to their sizes that constituted the well (Failure) from which the organoid-tissue module was not produced are shown in Table 5 below and Figure 13.

**[Table 5]**

| | Spheroid diameter (µm) | Average occupied space (%) |
|---|---|---|
| d1 | 100 or less | 1 |
| d2 | 100 to 200 | 5 |
| d3 | 200 to 300 | 69 |
| d4 | 300 to 400 | 25 |
| d5 | 400 to 500 | 0 |

As shown in Tables 4 and 5 above, when the spheroid diameters mixed in the organoid-tissue module initiation stage were of various sizes, from 50 to 500 µm, it was confirmed that organoid-tissue modules were not produced when the space occupied by spheroids having a diameter of 200 µm or less accounted for 6% of the space occupied by the total spheroids, and that organoid-tissue modules were produced when the space occupied by spheroids having a diameter of 200 µm or less accounted for 48% of the space occupied by the total spheroids.

In addition, it was confirmed that organoid-tissue modules were not produced when the space occupied by spheroids having a diameter of more than 200 µm accounted for 94% of the space occupied by the total spheroids, and that organoid-tissue modules were produced when the space occupied by spheroids having a diameter of more than 200 µm accounted for 52% of the space occupied by the total spheroids.

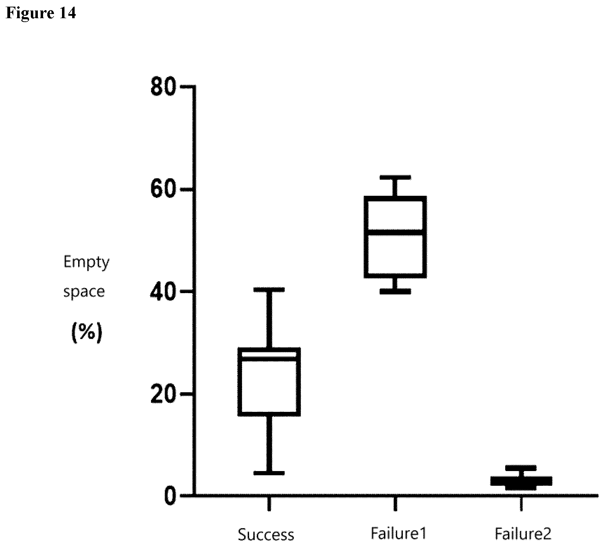

When the bottom area of the well was assumed to be 100%, the empty space (Remaining Space) excluding the space occupied by the spheroids was additionally analyzed using ImageJ software, and the results are shown in Figure 14 and Table 6.

As shown in Figure 14, Table 6, and Table 7, it was confirmed that the area of the empty space relative to the total area when the organoid-tissue modules were produced was 5 to 40%.

Ultimately, it can be seen that in order to produce organoid-tissue modules, the area of empty space is 5 to 40%, preferably 10 to 35%, and more preferably 15 to 30%.

**[Table 6]**

| | Success |
|---|---|
| Ratio of area not occupied by spheroids relative to culture area (%) | 5.895642 |
| | 6.08849 |
| | 6.722133 |
| | 11.5066 |
| | 14.49116 |
| | 15.20745 |
| | 16.429 |
| | 16.429 |
| | 16.78697 |
| | 19.28481 |
| | 21.47 |
| | 22.57241 |
| | 26.86098 |
| | 26.861 |
| | 27.182 |
| | 27.182 |
| | 27.908 |
| | 28.018 |
| | 28.734 |
| | 29.21189 |
| | 29.212 |
| | 30.06594 |
| | 30.066 |
| | 34.501 |
| | 34.575 |
| | 34.575 |
| Average | 22.60023 |
| Standard deviation | 9.838078 |

**[Table 7]**

| | Failure |
|---|---|
| Ratio of area not occupied by spheroids relative to culture area (%) | 1.689717 |
| | 1.864198 |
| | 2.314177 |
| | 2.800889 |
| | 3.315151 |
| | 3.636564 |
| | 3.783496 |
| | 42.53678 |
| | 44.28159 |
| | 51.57309 |
| | 52.5 |
| | 58.6 |
| | 62.3 |

### [Example 11]

### Bio-kinematic analysis of organoid-tissue module production

In order to confirm the self-assembly process of organoid-tissue modules, bio-kinematic analysis was performed as follows.

During the formation process of the organoid-tissue module, the movement of the spheroids was captured as an image, and based on the images, the spatiotemporal distribution of the spheroids, such as their speed, trajectory, linearity, and cooperativeness, was analyzed and calculated. Images captured over time were used to calculate spheroid motility within a limited space while the organoid-tissue modules were produced using the Image J program and a MATLAB code based on the particle image velocimetry (PIV) algorithm.

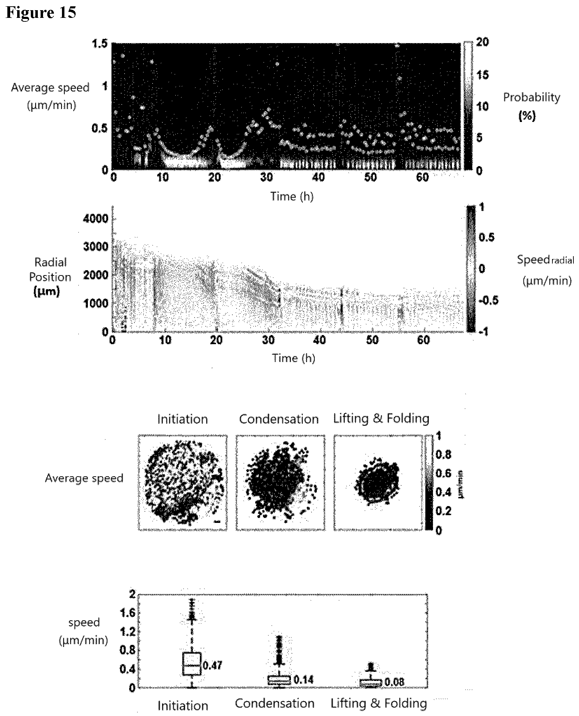

As shown in Figure 15, typical characteristics of spheroid movement were found in the initiation stage, condensation stage, and lifting and folding stage of organoid-tissue module generation. The average speed of spheroids fluctuated every 12 hours due to the exchange of culture medium every 12 hours, so the change in motility due to medium exchange was excluded from the analysis.

In the initiation phase, each spheroid exhibited irregular and different motility at different speeds (0-1.5 µm/min).

In the condensation stage, it was confirmed that the movement of the center of the organoid-tissue module was relatively static, while the spheroids in the boundary region still showed dynamic motility.

In the lifting and folding stage, the movement of most spheroids was reduced and stabilized, and only a slight repetitive movement was observed in the boundary region.

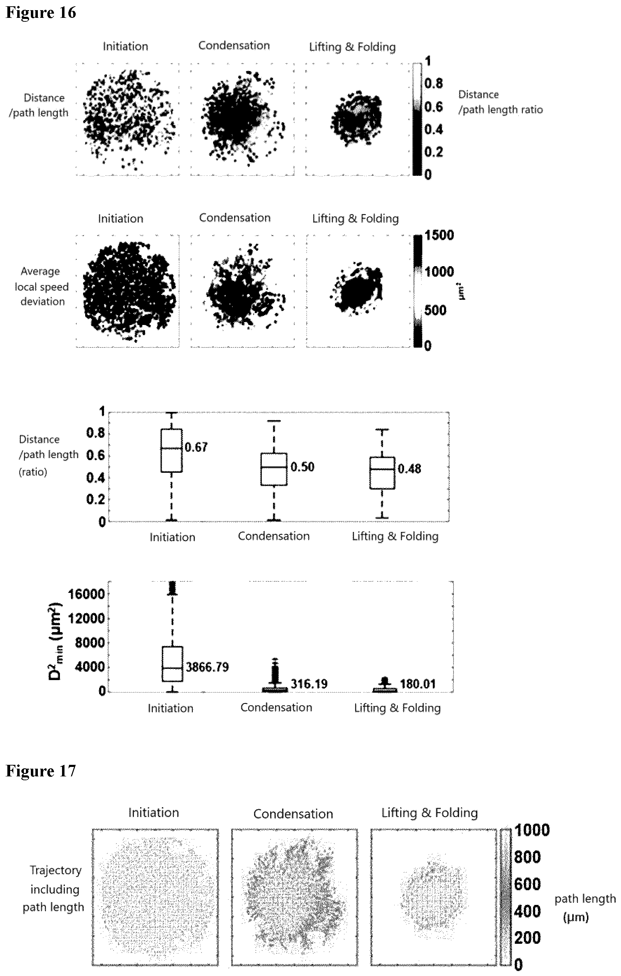

In addition, as shown in Figure 16, according to the analysis of the movement distance (path length) of the spheroids, the organoid-tissue module in the boundary region in the condensation stage showed a linear movement, while the spheroids in the central region showed a twisted movement. In the lifting and folding stage, the movement of the spheroids in the boundary region was hardly observed.

According to the trajectory analysis of Figure 17, the spheroids in the initiation stage showed random movement, but in the condensation stage, the spheroids in the boundary region were observed to move linearly toward the center. Interestingly, a longer trajectory and higher straightness were observed in the spheroids in the folding region than in the spheroids in the center. This movement is a phenomenon that generally occurs when aggregates form a stable structure, indicating that the organoid-tissue module in the lifting and folding stage has been converted to a structurally stable structure.

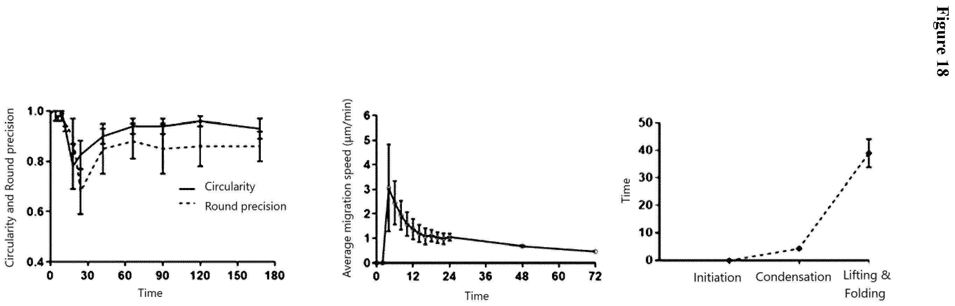

Additionally, as a result of analyzing phenotypic parameters such as circularity, as shown in Figure 18, after mixing spheroids and culturing for 24 hours, the circularity of the organoid-tissue module was temporarily reduced due to the motility of the spheroids, and then approached 1.0, which means a circle having the same diameter in all directions, after an additional 18 hours of culturing. Theses results indicate that the organoid-tissue module having an irregular structure begins to reorganize into a regular structure within 24 hours.

In addition, as shown in Figure 18, through bio-kinematic and morphological analyses, it can be seen that the organoid-tissue module self-organizes according to each stage of initiation stage (0 hours), condensation stage (4.3 hours), and lifting and folding stage (39 hours).

### [Example 12]

### Production of organoid-tissue modules using optimized conditions

From the experimental results of Examples 8 to 11 above, it was confirmed that the mixing ratio of spheroids with a diameter of 200 µm or less and spheroids with a diameter of more than 200 µm, the space occupancy ratio of spheroids with a diameter of 200 µm or less and spheroids with a diameter of more than 200 µm, and the area of empty space in the space where the organoid-tissue module is cultured are directly related to the production of the organoid-tissue module.

Therefore, experiments were performed under various conditions to confirm the above conditions.

Each microwell of the AggreWell plate was configured to accommodate 100, 500, 1000, 3000, and 5000 cells, and the culture solution used was DMEM containing 40 µg/ml L-proline, 100 µg/ml sodium pyruvate, 50 µg/ml L-ascorbic acid 2-phosphate, 1xITS, and 1xAA.

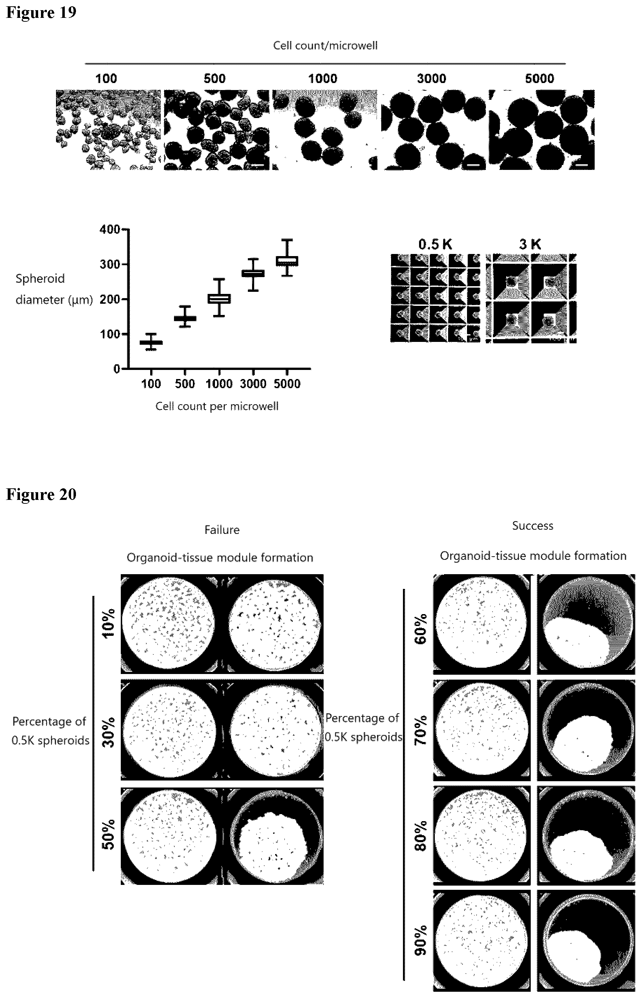

The results of culturing in Aggrewell are shown in Figure 19. Among the spheroids with an average diameter of 200 µm or less, spheroids composed of 500 cells were selected, and among the spheroids with an average diameter of more than 200 µm, spheroids composed of 3000 cells were selected. The spheroid composed of 500 cells was referred to as 0.5K spheroid, and the spheroid composed of 3000 cells was referred to as 3K spheroid.

As described in Example 10 above, the obtained 0.5K and 3K spheroids of two sizes were mixed and arranged in an ultra-low attachment 96-well plate with different numbers so that the empty area was about 20% of the total culture area.

First, when the 0.5K spheroids were increased to 10%, 30%, and 50%, self-assembly did not occur. On the other hand, when 0.5K spheroids and 3K spheroids were mixed at 60% or more of 0.5K spheroids and 40% or less of 3K spheroids, it was confirmed that organoid-tissue modules were formed through self-assembly of the spheroids, as shown in Figure 20. Therefore, it can be seen that when spheroids with different diameters are mixed and cultured, organoid-tissue modules are produced, and when the number of spheroids with a diameter of 200 µm or less is greater than the number of spheroids with a diameter of more than 200 µm, organoid-tissue modules are well produced.

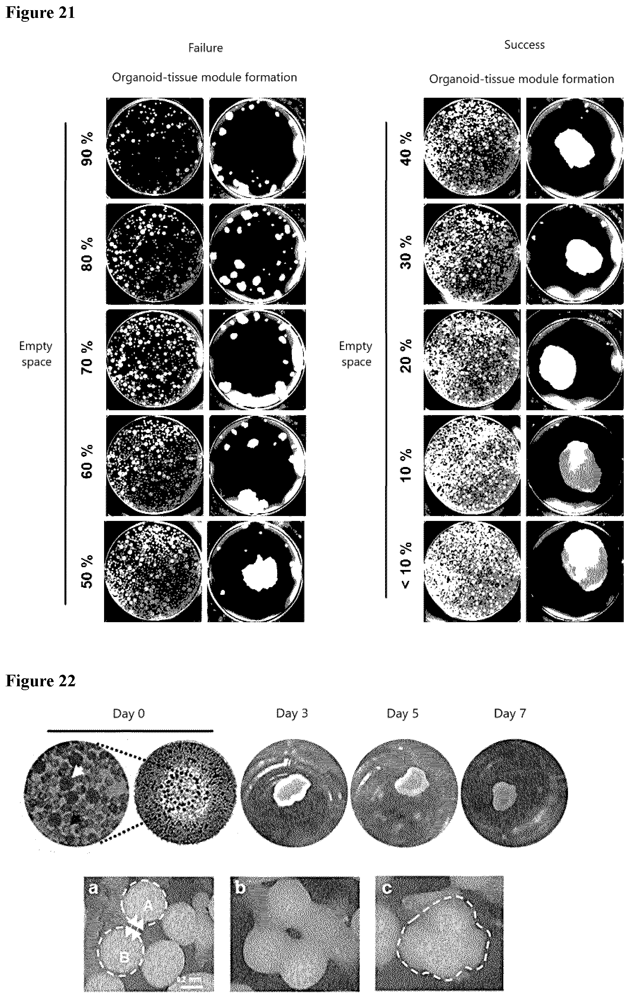

In order to investigate the effect of empty space on the formation of organoid-tissue modules, the mixing ratio of 0.5K spheroids and 3K spheroids was fixed at 90% and 10%, respectively, and they were mixed and arranged so that the empty culture areas that were not occupied by spheroids were 90%, 80%, 70%, 60%, 50%, 40%, 30%, 20%, 10%, and less than 10%, respectively, as shown in Figure 21. It was confirmed that when the empty area was greater than 50%, the self-assembly of the spheroid did not occur well, and when it was 40% or less, the organoid-tissue module was well formed by the self-assembly of the spheroid.

In another example, after the empty space area was 20% of the total culture area, the self-organization of the spheroids was induced through culture to produce an organoid-tissue module. As shown in Figure 22, the organoid-tissue module was generated through the self-assembly process, and it was confirmed that the cup shape was still maintained even after 7 days of culture.

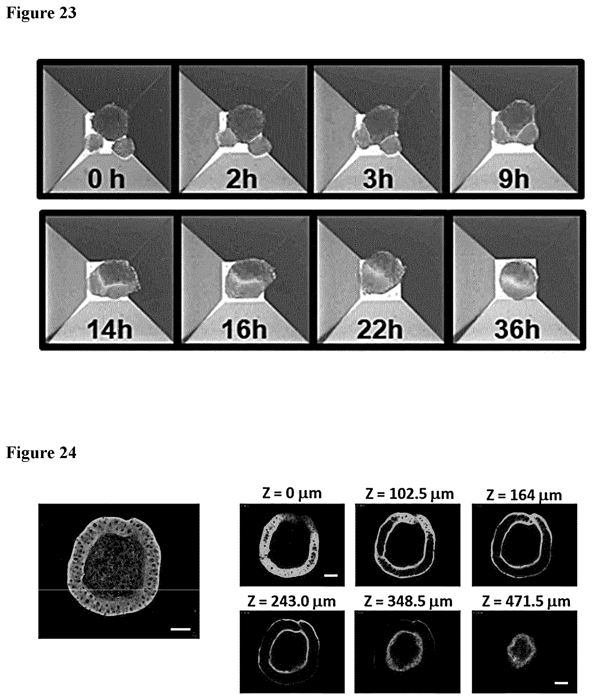

In addition, while the organoid-tissue module was generated, the spheroids were fused and condensed with adjacent spheroids to form a large structure. It was confirmed through the process of fusion of stained spheroids, when spheroids are fused to form organoid-tissue modules, the cells that make up the spheroids are not evenly mixed when different spheroids are fused, but are located in the original spheroids, as shown in Figure 23. After epifluorescence staining, the cup shape of the organoid-tissue modules was confirmed using a confocal microscope, as shown in Figure 24. The average diameter of the organoid-tissue modules was about 2 to 4 mm (10 to 35% of the culture area), which was confirmed to be about half the size of the pineal gland (5-8 mm), the smallest organ in the human body.

### [Example 13]

### Confirmation of stem cell characteristics of hMSCs of the produced organoid-tissue module

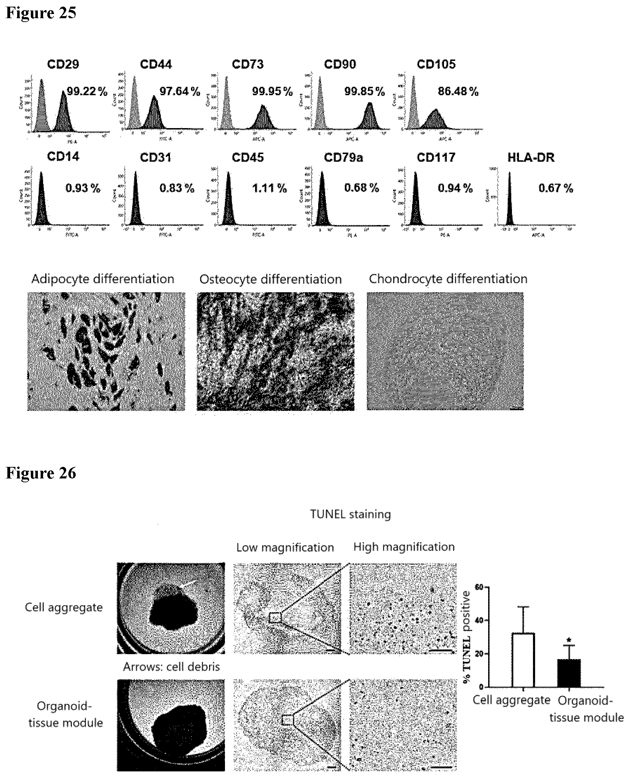

In order to confirm whether the hMSCs constituting the produced organoid-tissue module maintain the characteristics as stem cells, single cells were isolated from the organoid-tissue module and the expression of stem cell markers and differentiation ability were confirmed. As shown in Figure 25, it was confirmed that the cells constituting the organoid-tissue module express stem cell markers and differentiate into adipocytes, osteocytes, and chondrocytes, and maintain the characteristics as stem cells.

### [Example 14]

### Confirmation of apoptosis resistance of produced organoid-tissue modules

In order to confirm whether the produced organoid-tissue modules are more resistant to apoptosis than spherical cell aggregates, spherical aggregates and organoid-tissue modules composed of the same number of cells were produced. The spherical cell aggregates were prepared by placing the cells in a U-bottom plate, centrifuging at 500xg for 5 minutes, and then inducing aggregation in a 37°C incubator, and culturing for a total of 6 days under the same conditions as the culture conditions of the organoid-tissue modules. In the case of the spherical cell aggregates, there were many dead cell fragments around the spheroids due to apoptosis, but as shown in Figure 26, relatively very few dead cell fragments were observed in the organoid-tissue modules. The organoid-tissue modules and spherical cell aggregates were fixed in 4% PFA for one day and embedded in paraffin blocks. The slides were prepared at a thickness of 4 µm, deparaffinized, and treated with proteinase K to dissolve proteins in the tissue. Endogenous peroxidase was removed by treatment with 3% hydrogen peroxide (H₂O₂), and TUNEL (Terminal Deoxynucleotidyl Transferase dUTP Nick end Labeling) staining was performed using the ApoTag Peroxidase In Situ Apoptosis Detection Kit (Millipore). Images were acquired after counterstaining the nucleus with methyl green. As shown in Figure 26, no increased apoptosis was observed in the center of the organoid-tissue modules compared to the periphery. However, in the center of the spherical cell aggregates, there were many dead cells stained with TUNEL, and empty spaces were created due to necrosis. From the above, it is judged that the organoid-tissue module has a smooth supply of nutrients and oxygen due to its structural feature of a concave cup shape in the center, and it is judged that it has a technical advantage that can overcome the disadvantage of increasing apoptosis in the center as the size increases.

### [Example 15]

### Confirmation of possibility of differentiation into chondrocytes of produced organoid-tissue module through confirmation of type 2 collagen expression

The hMSCs used in the present invention are stem cells that can differentiate into chondrocytes, so they have been used as a promising cell source in cartilage tissue engineering (Somoza et al., 2014). In order to confirm the possibility of differentiation of the produced organoid-tissue module into chondrocytes, the differentiation of the organoid-tissue module into chondrocytes was induced by treatment with TGF-β3, and then the samples were recovered, fixed in 4% PFA for approximately 3 days, and immunohistochemistry (IHC) staining was performed for histological analysis.

To this end, the fixed sample was embedded in a paraffin block, and slides were prepared with a thickness of 4 µm. The slides were deparaffinized in histo-clear for 30 minutes, hydrated from 100% ethanol to 70% ethanol, and then washed with PBS. Thereafter, blocking was performed for 1 hour at room temperature with a PBS solution containing 0.3% bovine serum albumin (BSA), and then the primary antibody for type 2 collagen was diluted in the same solution at an appropriate concentration and left to stand at 4 °C for more than 12 hours. After the standing was completed, the secondary antibody was diluted in an appropriate concentration in a blocking solution after washing with PBS again, left to stand at 37 °C for 1 hour, washed with PBS, and reacted with DAB to develop color.

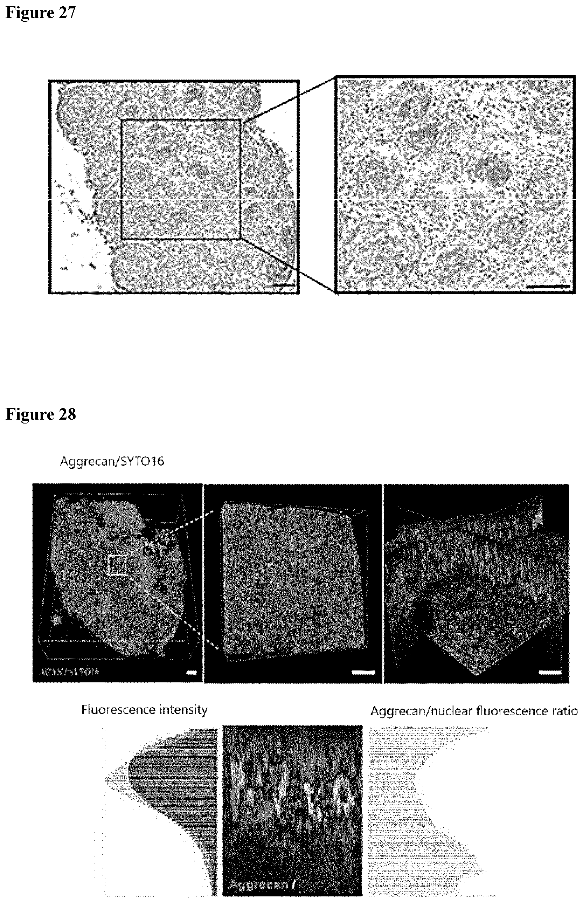

The results of the above experiment are shown in Figure 27, and it was confirmed that type 2 collagen was mainly expressed in spherical cells corresponding to large spheroids.

### [Example 16]

### Confirmation of possibility of differentiation of produced organoid-tissue module into chondrocytes through confirmation of Aggrecan

In order to confirm aggrecan, a marker of chondrocyte differentiation, a 3D clearing process was performed according to the method of Korean Patent No. 10-2170076 (Method for preparing decellularized tissue using hydrogel polymer and decellularized tissue prepared therefrom) to make the spheroid transparent, and then the expression of aggrecan was investigated in 3D using an antibody.

To this end, the produced organoid-tissue module was fixed in 4% PFA for 1 week and washed with PBS containing 0.1% triton-X, and then a blocking process was performed at room temperature for at least 8 hours using a solution containing 6% BSA, 0.2% triton X-100, and 0.01% sodium azide in PBS. Thereafter, the aggrecan antibody was diluted to an appropriate concentration in the blocking solution, dispensed into the organoid-tissue module, and reacted at room temperature for 2 days. Washing was performed with the previously used washing solution, and the secondary antibody and hoechest were diluted to an appropriate concentration in the blocking solution, dispensed into the spheroid, and reacted at room temperature for 2 days. After the antibody reaction was completed through the washing process, a transparentization process was performed using a solution containing 25% urea and 65% sucrose in water.

The results of the above experiment are shown in Figure 28, and it was confirmed that the expression of aggrecan was highest at the edge of the organoid-tissue module.

### [Example 17]

### Quantitative comparative analysis of cartilage differentiation through glycosaminoglycan

A GAG assay was performed for quantitative comparative analysis of cartilage differentiation through glycosaminoglycan (GAG), an important extracellular matrix of cartilage.

To this end, 7.8 µL of papain was added to a solution containing 200 mM sodium phosphate, 100 mM sodium acetate, 10 mM ethylene diamine tetraacetic acid (EDTA), and 5 mM L-cysteine, and adjusted to pH 6.4 to prepare a papain solution, and the sample was added and reacted in a 65°C constant temperature water bath for 18 hours. After 18 hours, centrifugation was performed at 10,000 g for 10 minutes, and the GAG content was measured according to the instructions of the sulfate glycosaminoglycan assay kit.

The absorbance of the sample was measured at 656 nm using a microplate reader, and the total DNA amount was quantified using a pico-green dsDNA assay kit to normalize the measurement results.

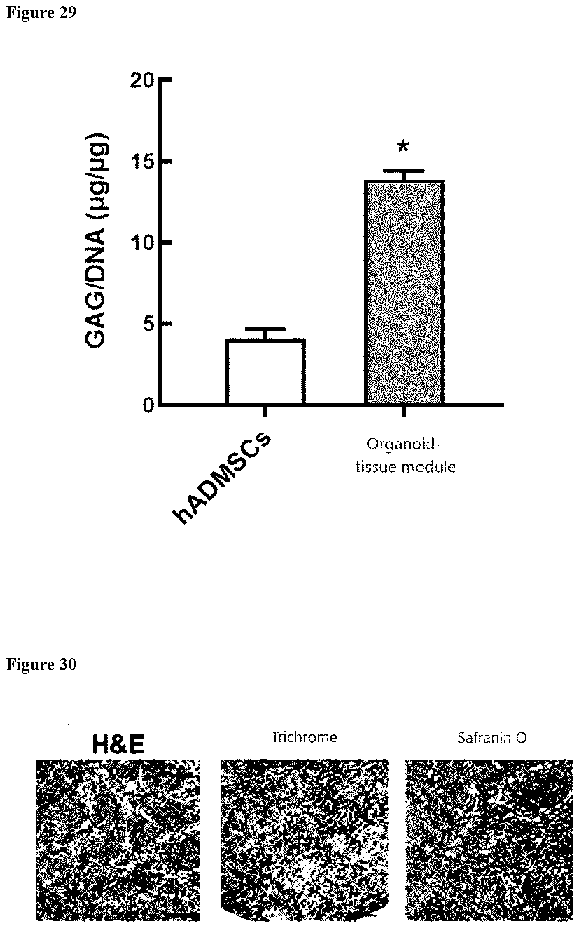

The results of the above experiment are shown in Figure 29, and it was confirmed that the GAG content was significantly increased in the organoid-tissue module compared to the single cell.

### [Example 18]

### Histological analysis of organoid-tissue module in which cartilage differentiation is induced using various staining techniques

In order to confirm the results of immunohistological staining, the organoid-tissue module sample was embedded in a paraffin block and cut into slides with a thickness of 4 µm. After deparaffinization was performed using the same method as above, it was stained using a hematoxylin & eosin (H&E) solution, and the synthetic collagen in the organoid-tissue module was detected using a Trichrome III Blue Staining Kit, and GAG expression was visualized using a Safranin-O/fast green kit.

The results of the above experiment are shown in Figure 30. As a result of Masson's trichrome staining, it was confirmed that the entire area was stained blue due to collagen synthesis, and as a result of Safranin-O staining, it was confirmed that GAG, which was stained red, was expressed in all locations of the organoid-tissue module. Therefore, it was confirmed that the organoid-tissue module was differentiated into chondrocyte organoids by cartilage differentiation factors such as TGF-β3.

### [Example 19]

### Confirmation of efficacy of organoid-tissue module through cartilage defect rabbit model

In order to confirm the cartilage regeneration efficacy of organoid-tissue module for cartilage defect osteoarthritis (CD/osteoarthritis (OA)) model, a cartilage defect model was produced in which the entire cartilage layer was removed from the knee of adult New Zealand white rabbits, and an experiment was conducted to transplant the organoid-tissue module into the defect site. The experiment was conducted with the approval of the Institutional Animal Care and Use Committee of Seoul National University Hospital (SNUH-IACUC No. 18-0171-S1A0), and the animals were maintained in a facility assessed and accredited by the Association for Assessment and Accreditation of Laboratory Animal Care (AAALAC) in accordance with the 8th edition of the Guide for the Care and Use of Laboratory Animals of the National Research Corporation (NRC).

Rabbit was anesthetized by intramuscular injection with a mixture of 5 mg/kg Xylazine and 15 mg/kg Zoletil, and a cartilage defect measuring 3 mm in diameter x 3 mm in depth was induced in the knee joint using sterile surgical instruments. The organoid-tissue module transplant group was sutured after inserting the organoid-tissue module, and 0.3 mL of Metacam and 1 mL of Cefazolin were injected subcutaneously for antibiotics and analgesics treatment for 3 days after surgery. The animals were sacrificed 12 or 14 weeks after surgery, and the knees were cut and micro-computed tomography (Micro-CT) was taken before histological evaluation.

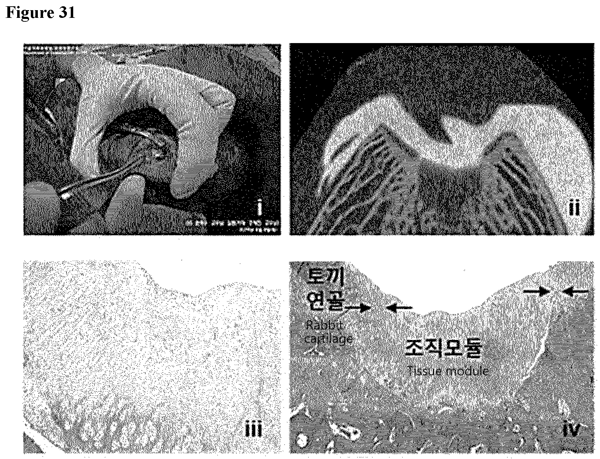

As a result of taking pictures of rabbit cartilage using Micro-CT after the experiment, it was confirmed that all the defected areas were completely filled, as shown in Figure 31. In addition, paraffin sections were prepared using rabbit knee cartilage tissue and H&E staining was performed. As a result, it was confirmed that the generation of the epiphyseal plate structure was increased in the defected cartilage, and a wide range of extracellular matrix and osteophyte morphology were clearly confirmed at the transplantation site of the organoid-tissue module.

In addition, as a result of confirming the relevant site through safranin-O staining, as shown in Figure 31, the formation of hyaluronic cartilage was confirmed in the organoid-tissue module transplantation group.

Through the above experiment, it was confirmed that the cartilage generated 12 weeks after transplantation of the organoid-tissue module into the CD/OA rabbit model was thicker than the existing surrounding cartilage of the rabbit, and the organoid-tissue module was completely fused with the damaged cartilage. Ultimately, it was found that the organoid-tissue module produced in millimeter size had an excellent regenerative effect in the CD/OA model.

## Claims

1. A method for preparing an organoid-tissue module by mixing and culturing spheroids with different diameters.

2. The method for preparing an organoid-tissue module according to claim 1, wherein the spheroid has a diameter of 10 to 1,000 µm.

3. The method for preparing an organoid-tissue module according to claim 1, wherein the spheroid has a diameter of 50 to 500 µm.

4. The method for preparing an organoid-tissue module according to claim 1, wherein spheroids having a diameter of 200 µm or less account for 60% or more of the total spheroids.

5. The method for preparing an organoid-tissue module according to claim 1, wherein spheroids having a diameter of 200 µm or less account for 70% or more of the total spheroids.

6. The method for preparing an organoid-tissue module according to claim 1, wherein spheroids having a diameter of 200 µm or less account for 80% or more of the total spheroids.

7. The method for preparing an organoid-tissue module according to claim 1, wherein spheroids having a diameter of more than 200 µm account for 40% or less of the total spheroids.

8. The method for preparing an organoid-tissue module according to claim 1, wherein spheroids having a diameter of more than 200 µm account for 30% or less of the total spheroids.

9. The method for preparing an organoid-tissue module according to claim 1, wherein spheroids having a diameter of more than 200 µm account for 20% or less of the total spheroids.

10. The method for preparing an organoid-tissue module according to claim 1, wherein the number of spheroids having a diameter of 200 µm or less is greater than the number of spheroids having a diameter of more than 200 µm.

11. The method for preparing an organoid-tissue module according to claim 1, wherein the space not occupied by the spheroids accounts for 5 to 40% of the total space for culturing the spheroids.

12. The method for preparing an organoid-tissue module according to claim 1, wherein the space not occupied by the spheroids accounts for 10 to 35% of the total space for culturing the spheroids.

13. The method for preparing an organoid-tissue module according to claim 1, wherein the space not occupied by the spheroids accounts for 15 to 30% of the total space for culturing the spheroids.

14. The method for preparing an organoid-tissue module according to claim 1, wherein the tissue module occupies 10 to 35% of the culture area.

15. The method for preparing an organoid-tissue module according to claim 1, wherein the tissue module has a diameter of about 2 to 4 mm.

16. The method for preparing an organoid-tissue module according to claim 1, wherein the spheroid is obtained by culturing stem cells or undifferentiated cells.

17. The method for preparing an organoid-tissue module according to claim 16, wherein the stem cell or undifferentiated cell is selected from the group consisting of an embryonic stem cell, a dedifferentiated stem cell, an adult stem cell, and a mesenchymal stem cell.

18. The method for preparing an organoid-tissue module according to claim 17, wherein the stem cell is a human adipose-derived mesenchymal stem cell (hMSC).

19. The method for preparing an organoid-tissue module according to claim 1, wherein the organoid-tissue module has a cup-shaped morphology.

20. The method for preparing an organoid-tissue module according to claim 1, wherein the method does not include a scaffold and an artificial material.

21. An organoid-tissue module prepared by the preparation method according to any one of claims 1 to 20.

22. A pharmaceutical composition for preventing or treating cartilage disease, comprising the organoid-tissue module according to claim 21.
